**Europäisches Patentamt**

**European Patent Office**

**Office européen des brevets**

(19)

(11) Veröffentlichungsnummer : **0 469 573 B1**

(12)

# EUROPÄISCHE PATENTSCHRIFT

(45) Veröffentlichungstag der Patentschrift :
**29.12.93 Patentblatt 93/52**

(51) Int. Cl.$^5$ : **A61K 6/02,** C08L 33/02, C08K 3/40

(21) Anmeldenummer : **91112867.6**

(22) Anmeldetag : **31.07.91**

(54) **Verformbare Masse und deren Verwendung als Füllungsmaterial für Zahnwurzelkanäle.**

(30) Priorität : **31.07.90 DE 4024322**

(43) Veröffentlichungstag der Anmeldung :
**05.02.92 Patentblatt 92/06**

(45) Bekanntmachung des Hinweises auf die
Patenterteilung :
**29.12.93 Patentblatt 93/52**

(84) Benannte Vertragsstaaten :
**AT CH DE ES FR GB IT LI**

(56) Entgegenhaltungen :
**EP-A- 0 329 268**
**DE-A- 3 806 448**
**DE-A- 3 825 027**
**DE-A- 3 903 703**
**DE-B- 2 319 715**

(73) Patentinhaber : **THERA Patent GmbH & Co. KG**
**Gesellschaft für industrielle Schutzrechte**
**Am Griesberg 2**
**D-82229 Seefeld (DE)**

(72) Erfinder : **Gasser, Oswald, Dr.**
**Höhenstrasse 10**
**W-8031 Seefeld (DE)**
Erfinder : **Guggenberger, Rainer, Dr.**
**Siglstrasse 10**
**W-8036 Herrsching (DE)**

(74) Vertreter : **Abitz, Walter, Dr.-Ing. et al**
**Patentanwälte Abitz & Partner Postfach 86 01 09**
**D-81628 München (DE)**

EP 0 469 573 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Aufgrund von Karies, undichten Füllungen, traumatischen Prozessen und auf anderen Infektionswegen kann sich die Pulpa (d.h. das Zahnmark) des lebenden Zahnes entzünden. Diese Entzündung ist in den meisten Fällen irreversibel, so dass die Pulpa durch den Zahnarzt entfernt werden muss. Ausser der Pulpa ist auch das angrenzende, infizierte Hartgewebe sorgfältig zu entfernen, wonach der gebildete Hohlraum - der Wurzelkanal - wieder dicht verschlossen werden muss. Zu diesem Zweck sind eine Reihe von Produkten auf dem Markt, die im wesentlichen aus Zinkoxid oder Calciumhydroxid zusammen mit einem organischen Salzbildner bestehen. Keines der Materialien erfüllt jedoch vollständig die Anforderungen an ein derartiges Wurzelkanalfüllmaterial.

Dieses Material muss den Kanal dicht verschliessen, um das Eindringen infektiöser Keime - es besteht die Gefahr der Kieferhöhleninfektion - zu unterbinden. Eine hohe Gewebeverträglichkeit ist zu fordern, da der Zement beim Austreten aus dem Apex (Zahnwurzelspitze) mit lebendem Gewebe in Berührung kommen kann. Ausserdem darf der Zement zur Erhaltung der Dichtheit nur eine sehr geringe Löslichkeit aufweisen. Schliesslich sind eine ausreichende Verarbeitungszeit sowie eine geeignete Viskosität für das Einbringen in den engen Wurzelkanal vonnöten.

Um die Dichtigkeit der Füllungsmaterialien zu erhöhen und um ihre Schrumpfung bei der Erhärtungsreaktion auszugleichen, wird das Material häufig nur als Versiegelung zusammen mit dünnen Stiften aus Guttapercha (einem gefüllten, natürlichen Harzmaterial) verwendet. Nach einer lehrbuchmässigen Methode werden dabei viele derartige Stifte lateral kondensiert, um den Anteil an Füllungsmaterial möglichst gering zu halten. Diese Methode ist jedoch enorm zeitaufwendig.

Glasionomerzemente sind bekannt für ihren dichten Randschluss, da sie an der Zahnsubstanz chemisch-physikalische Haftung aufweisen; ausserdem zeigen sie nur sehr geringe Löslichkeit und werden als gewebeverträglich eingestuft. Jedoch mangelt es ihnen an einer ausreichenden Verarbeitungszeit zum Einbringen in den Wurzelkanal. Bei den herkömmlichen Anwendungsbereichen für Glasionomerzemente - Zahnfüllungen, Befestigungszementen, Versiegelungsmaterialien, Stumpfaufbaumaterialien - beträgt die benötigte Verarbeitungszeit nur 2 - 3 Minuten. Für die Wurzelkanalfüllung einschliesslich der erforderlichen Röntgenkontrolle benötigt der Zahnarzt jedoch ca. 10 Minuten Zeit. Weiterhin ist die Viskosität der Glasionomerzemente zu hoch - d.h. die Pasten sind zu fest - um sie mit den herkömmlichen Mitteln in den Wurzelkanal einzubringen. Ein Verdünnen des Systems führt jedoch zu einer viel zu hohen Löslichkeit.

Aus der US-Patentschrift 4 861 808 sind Glasionomer-Zusammensetzungen bekannt, die ein Leichtmetallfluorid, nämlich Strontiumfluorid, enthalten, um aus diesen Zusammensetzungen hergestellte Zahnfüllungen röntgensichtbar zu machen. Die Röntgenopazität und die Verarbeitbarkeit dieser bekannten Zusammensetzungen sind jedoch nicht zufriedenstellend.

Die US-Patentschrift Re. 33 100 beschreibt Glasionomer-Zusammensetzungen, die zur Vermeidung einer Zahnpulpairritation mit 5 bis 20 Gew.-% eines löslichen Schwermetalloxides, nämlich Zinkoxid, und 0 bis 10 Gew.-% Titandioxid gepuffert sind. Die Verarbeitbarkeit dieser Zusammensetzungen und die Beständigkeit von aus diesen Zuammensetzungen hergestellten Zahnfüllungen sind nicht zufriedenstellend.

Aufgabe der Erfindung ist die Bereitstellung eines Wurzelkanalfüllmaterials, welches in möglichst idealer Weise den klinischen Anforderungen gerecht wird, d.h. eine ausreichende Verarbeitungszeit aufweist und dichte Füllungen ergibt, die eine hohe mechanische Festigkeit und eine geringe Löslichkeit aufweisen.

Überraschenderweise wurde festgestellt, dass durch Zusatz bestimmter Schwermetallverbindungen zu einem herkömmlichen Glasionomerzement ein geeignetes Wurzelkanalfüllmaterial erhalten wird.

Die Erfindung betrifft eine verformbare Masse, bestehend aus

(A) 25 bis 80 Gew.-% eines Glasionomerzementes, enthaltend

    (a) ein Aluminiumfluorosilikatglas,

    (b) mindestens eine polymere Polysäure mit einem mittleren Molekulargewicht > 500,

    (c) Wasser und

    (d) gegebenenfalls ein chelatbildendes Mittel, und

(B) 20 bis 75 Gew.-% eines in der wässrigen polymeren Polysäure und in der den Chelatbildner enthaltenden Lösung im wesentlichen unlöslichen Fluorids und/oder Oxids und/oder Mischfluorids und/oder Mischoxids von Schwermetallelementen.

Vorzugsweise liegen die Komponenten (A) und (B) jeweils in Mengen von 35 bis 65 Gew.-%, insbesondere 50 bis 65 Gew.-%, vor.

Überraschenderweise sind trotz des hohen Zusatzes an inerter Schwermetallverbindung, d.h. trotz deutlicher Verringerung des reaktiven Glasanteils, relativ gute mechanische Festigkeit und vor allem geringe Löslichkeit vorhanden, wobei die Mischung eine für die Applikation gut geeignete Fliessfähigkeit aufweist. Zusätzlich erhöht sich die Verarbeitungszeit für die plastische Masse in der gewünschten Weise, ohne dass nennenswerte Zugeständnisse bei der Löslichkeit und der mechanischen Festigkeit gemacht werden müssen.

Als Bestandteil (A)(a) können die in der DE-A-20 61 513 und der EP-A-0 023 013 beschriebenen Calciumaluminiumfluorosilikatgläser und die in der EP-A-0 241 277 beschriebenen Strontiumaluminiumfluorosilikatgläser eingesetzt werden. Die erfindungsgemäss verwendeten Aluminiumfluorosilikatglas-Pulver bestehen neben Sauerstoff vorzugsweise aus:

| Bestandteil | Berechnet als | Gew.-% |
|---|---|---|
| Si | $SiO_2$ | 10 – 60 |
| Al | $Al_2O_3$ | 10 – 50 |
| Ca | CaO | 0 – 40 |
| Sr | SrO | 0 – 40 |
| F | F | 1 – 40 |
| Na | $Na_2O$ | 0 – 10 |
| P | $P_2O_5$ | 0 – 10 |

wobei mindestens 1 Gew.-% CaO und/oder SrO enthalten sein müssen und insgesamt 0 bis 30 Gew.-%, berechnet als Oxide, an B, Bi, Zn, Mg, Sn, Ti, Zr, La oder anderen 3-wertigen Lanthanoiden, K, W, Ge sowie weiteren Zusätzen, welche die Eigenschaften nicht beeinträchtigen und physiologisch unbedenklich sind. Durch Zusatz von 10 bis 30 Gew.-% $La_2O_3$ können die Gläser röntgensichtbar gemacht werden.

Vorteilhaft bestehen die Pulverteilchen aus:

Si als $SiO_2$      20 - 50 Gew.-%
Al als $Al_2O_3$      10 - 40 Gew.-%
Ca als CaO      0 - 35 Gew.%
Sr als SrO      0 - 35 Gew.-%
F      5 - 30 Gew.%
Na als $Na_2O$      0 - 8 Gew.-%
P als $P_2O_5$      1 - 10 Gew.-%

wobei 0 bis 30 Gew.-% Ca (berechnet als CaO) und/oder Sr (berechnet als SrO) enthalten sein müssen und 0 - 10 Gew.-% an $B_2O_3$, $Bi_2O_3$, ZnO MgO, $SnO_2$, $TiO_2$, $ZrO_2$, $La_2O_3$ oder anderen Oxiden 3-wertiger Lanthanoide, $K_2O$, $WO_3$, $GeO_2$ sowie weiteren Zusätzen, welche die Eigenschaften nicht beeinträchtigen und physiologisch unbedenklich sind.

Besonders bevorzugt verwendete Pulver enthalten

Si als $SiO_2$      20 - 40 Gew.-%
Al als $Al_2O_3$      15 - 35 Gew.-%
Ca als CaO      5 - 25 Gew.%
F      10 - 30 Gew.-%
Na als $Na_2O$      1 - 8 Gew.-%
P als $P_2O_5$      1 - 10 Gew.-%
La als $La_2O_5$      0 - 30 Gew.-%

Die erfindungsgemäss verwendeten Glaspulver weisen eine durchschnittliche Korngrösse (Gewichtsmittel) von wenigstens 1 µm und bevorzugt mindestens 3 µm auf. Die durchschnittliche Korngrösse (Gewichtsmittel) beträgt 1 - 20 µm, bevorzugt 3 - 15 µm und besonders bevorzugt 3 - 10 µm. Die Teilchen haben eine maximale Korngrösse von 150 µm, vorzugsweise 100 µm, besonders 60 µm.

Die so erhaltenen Pulver werden dann gegebenenfalls einer Oberflächenbehandlung gemäss der europäischen Patentschrift 0 023 013 unterzogen. Hierzu werden die Glaspulver mit Säure oberflächlich behandelt, vorzugsweise bei Raumtemperatur. Dabei werden saure Gruppen enthaltende Substanzen eingesetzt, z.B. Salzsäure, Schwefelsäure, Salpetersäure, Essigsäure, Propionsäure oder Perchlorsäure, die lösliche Calciumsalze bzw. Strontiumsalze bilden. Die Säuren werden in einer Konzentration von 0,01 bis 10 Gew.-%, vorzugsweise von 0,05 bis 3 Gew.-% eingesetzt. Nach der entsprechenden Reaktionszeit werden die Pulver von der Lösung separiert und gründlich ausgewaschen, so dass sich praktisch keine löslichen Calcium- oder Strontiumsalze mehr auf der Oberfläche der Pulverteilchen befinden.

Die als Bestandteil (A)(b) einzusetzenden polymeren Polysäuren können auch die bei der Herstellung von Glasionomerzementpulvern bekannten Polycarbonsäuren, z.B. Polymaleinsäure, Polyacrylsäure, Polyitaconsäure sowie Gemische davon oder Copolymere, insbesondere die aus der EP-A-0 024 056 bekannten Maleinsäure-Acrylsäure-Copolymeren und/oder Acrylsäure-Itaconsäure-Copolymeren sein. Das mittlere Molekulargewicht der erfindungsgemäss einzusetzenden Polycarbonsäuren beträgt mehr als 500. Vorteilhaft ist ein mittleres Molekulargewicht von 1.000 bis 20.000, besonders bevorzugt sind 3.000 bis 10.000. Die Polysäure wird vorzugsweise in Konzentrationen von 5 bis 50 Gew.-%, bezogen auf Bestandteil (A)(a), eingesetzt.

Als polymere Polysäure geeignet sind auch Polyphosphonsäuren, z.B. Polyvinylphosphonsäure. Diese Polyphosphonsäuren können die oben genannten Polycarbonsäuren ganz oder teilweise ersetzen.

Der Bestandteil (A)(c), das Wasser, wird in Mengen von 5 bis 70 Gew.-%, vorzugsweise 15 bis 40 Gew.-%, bezogen auf die Gesamtmasse von (A), eingesetzt.

Als Bestandteil (A)(d) kann ein chelatbildendes Mittel, wie es in der DE-A-23 19 715 beschrieben ist, enthalten sein. Vorzugsweise wird als Chelatbildner Weinsäure eingesetzt. Die Chelatbildner können in Konzentrationen von 0,1 bis 10, vorzugsweise 3 bis 8 Gew.-%, bezogen auf die Gesamtmasse von (A), eingesetzt werden.

Um hohe Lagerstabilität vor der Anwendung zu erhalten, empfiehlt sich ein Zusatz von Konservierungsstoffen, z.B. Benzoesäure, insbesondere zur trockenen Polysäure.

Zusätze zur Einstellung der Viskosität (z. B. pyrogene Kieselsäure) und zur Einfärbung (Pigmente) sind möglich. Die Viskositätsregler können in Konzentrationen von 0,1 bis 10, vorzugsweise 1 bis 5 Gew.-%, bezogen auf das Gewicht der Gesamtmasse von (A), eingesetzt werden.

Die als Komponente (B) vorhandene Schwermetallverbindung ist physiologisch unbedenklich und inert, d.h. sie ist weitgehend unlöslich in den für Glasionomerzemente üblicherweise verwendeten polymeren Polysäuren, und sie ist ausgewählt aus der Gruppe der in der wässrigen polymeren Polysäure und in der den Chelatbildner enthaltenden Lösung im wesentlichen unlöslichen Fluoride und/oder Oxide und/oder Mischoxide und/oder Mischfluoride der Schwermetallelemente. Schwermetalle sind solche Elemente, die eine Dichte von etwa > 4 aufweisen. Bevorzugt sind Oxide/Fluoride der seltenen Erdmetalle und der 6. Nebengruppe. Besonders bevorzugt ist die Gruppe Yttriumfluorid, Zirkondioxid, Lanthanoxid, Calciumwolframat und Strontiumwolframat. Ganz besonders bevorzugt ist Calciumwolframat. Die mittlere Korngrössen liegen dabei zwischen 0,5 µm und 20 µm, vorzugsweise zwischen 2 µm und 10 µm.

Bevorzugt wird die erfindungsgemässe Masse in einer Kapsel angeboten, die Pulver und Flüssigkeit zunächst getrennt enthält. Nach Zusammenfügen von Pulver und Flüssigkeit wird die Masse durch mechanisches Anmischen mit einem üblichen Schüttelgerät zubereitet.

Vorzugsweise kommt eine Applikationskapsel nach EP-A-0 157 121 zur Anwendung, die besonders bevorzugt mit einem Kunststoffröhrchen gemäss DE-GM 90 03 983.1 versehen ist. Damit ist die Direktapplikation der angemischten, erfindungsgemässen Masse in den Wurzelkanal möglich.

Bevorzugt wird die Ausgangskomponente (Pulver und Flüssigkeit) steril bereitgestellt. Die Sterilisation kann dabei durch Hitzebehandlung, Sterilfiltration (Flüssigkeit) oder insbesondere durch Gammabestrahlung erfolgen.

Die Aufteilung der wesentlichen Bestandteile des Systems

(A)(a)     Glaspulver
(A)(b)     polymere Polysäure
(A)(c)     Wasser
(A)(d)     Chelatbildner
(B)        Schwermetallverbindung

kann auf verschiedene Weise erfolgen:

```
Pulver                              Flüssigkeit

(A)(a) + B                      -   (A)(b) + (A)(c) + (A)(d)

(A)(a) + B + (A)(b)             -   (A)(c) + (A)(d)

(A)(a) + B + (A)(b) + (A)(d)    -   (A)(c)
```

Möglich sind auch Paste/Paste-Aufteilungen, bei denen z.B. mit (A)(c) kombiniert werden könnte.

BEISPIELE

Beispiel 1

Es wird ein Zementpulver hergestellt durch inniges Vermischen von
- 75 g Calciumwolframat
- 25 g Calciumaluminiumfluorosilikatglas der folgenden Zusammensetzung:
    7 Gew.-% Aluminiumfluorid
    13 Gew.-% Aluminiumoxid
    12 Gew.-% Aluminiumphosphat
    20 Gew.-% Calciumfluorid
    4 Gew.-% Kryolith
    20 Gew.-% Lanthanoxid
    24 Gew.-% Quarz
    (mittlere Korngrösse 8 μm)
    welches eine Stunde mit der 10-fachen Menge 0,1 %-iger Salzsäure behandelt, getrocknet und
  6 Stunden bei 400°C erhitzt worden war.
- 4 g pyrogene Kieselsäure und
    Pigmenten.
Weiterhin wurde eine Zementflüssigkeit hergestellt durch Lösen von
35 g eines Copolymeren (1 : 1) aus Acrylsäure und Maleinsäure (mittleres Molgewicht ca. 8.000 Dalton) und
11,8 g Weinsäure in
53,2 g destilliertem Wasser.
300 mg des Zementpulvers werden mit
100 mg der Zementflüssigkeit mit einem Spatel homogen vermischt.

Die Viskosität der Mischung ist gut geeignet, um das Material mit dem Lentulo (spiralförmige Einbringvorrichtung) in den Wurzelkanal einzuführen. Die Röntgenkontrolle zeigt den eingefüllten Zement homogen, randdicht und blasenfrei. Der Zement ist gut zu verarbeiten und zeigt gute Haftung an der Zahnsubstanz. Die physikalischen Daten sind aus Tabelle 1 ersichtlich.

Beispiel 2

In dem Zementpulver nach Beispiel 1 wird das Calciumwolframat durch 65 g Zirkondioxid (mittlere Korngrösse 6 μm) ersetzt.

Die Weiterverarbeitung erfolgt wie in Beispiel 1 beschrieben. Der erhaltene Zement lässt sich einwandfrei verarbeiten und in den Wurzelkanal einbringen (physikalische Daten siehe Tabelle 1).

Beispiel 3

100 mg der Flüssigkeit aus Beispiel 1 werden in ein kleines Folienkissen eingeschweisst und an einer Applikationskapsel gemäss EP-A-0 157 121 angebracht, deren Hauptkammer mit 300 mg des Zementpulvers nach Beispiel 1 gefüllt ist. An der Spitze des beweglichen Rüssels ist ein Applikationsröhrchen nach DE-GM G 90 03 983.1 angebracht. Die Kapsel ist in eine Blisterfolie eingeschweisst und wird durch Gammastrahlen sterilisiert. Der Zement, der in seinen Eigenschaften dem Material nach Beispiel 1 entspricht, kann nach Anmischen in einem mechanischen Mischgerät (Capmix, Fa. ESPE) (Mischdauer 10 Sekunden) direkt in den Wurzelkanal appliziert werden.

Vergleichsbeispiel 1

Ein auf dem Markt befindliches Wurzelkanalfüllmaterial auf Basis Zinkoxid/Propionyl/Acetophenon (Diaket®, Fa. ESPE) wurde nach Herstellerangaben angemischt und verarbeitet (physikalische Daten siehe Tabelle 1).

Vergleichsbeispiel 2

Entsprechend Beispiel 1 wird ein Zement hergestellt. Im Unterschied dazu besteht das Pulver jedoch lediglich aus dem beschriebenen Calciumaluminiumfluorosilikatglas ohne die erfindungsgemässe Schwermetallverbindung (siehe Tabelle 1).

TABELLE 1 – Physikalische Eigenschaften

| | Beispiel 1 | Beispiel 2 | Vergleichs-beispiel 1 | Vergleichs-beispiel 2 |
|---|---|---|---|---|
| Verarbeitungszeit (23°C)[1] | 11 Min. | 9:30 Min. | 10 Min. | 4 Min. |
| Abbindezeit (36°C)[1] | 10:30 Min. | 10 Min. | 135 Min. | 4:30 Min. |
| Löslichkeit[2] | 0,4 % | 0,6 % | 0,5 % | 0,3 % |
| Druckfestigkeit[2] | 90 MPa | 82 MPA | <10 MPA | 95 MPA |
| Röntgensichtbarkeit[1] | 9 mm Al | 8 mm Al | 5 mm Al | 3 mm Al |
| Viskosität[1] | 32 mm | 33 mm | 32 mm | 25 mm |

1) Messung nach ADA Nr. 57
2) Messung nach ISO 7489

Der Vergleich der physikalischen Eigenschaften der Beispiele (Tabelle 1) lässt folgende Schlüsse zu:

Die erfindungsgemässen Wurzelfüllmaterialien zeigen eine lange Verarbeitungszeit, die durch einen Glasionomerzement ohne Zusatz (Vergleichsbeispiel 2) nicht erreicht werden kann. Dennoch ist die Abbindezeit relativ rasch. Sie ist wesentlich schneller als bei auf dem Markt befindlichen Wurzelkanalfüllmaterialien (Vergleichsbeispiel 1).

Trotz des hohen Gehaltes an inerter Schwermetallverbindung sind Löslichkeit und Druckfestigkeit gegenüber einem reinen Glasionomerzement (Vergleichsbeispiel 2) nahezu unverändert. Die Druckfestigkeit liegt deutlich über dem derzeitigen Niveau von bekannten Wurzelkanalfüllmitteln (Vergleichsbeispiel 1).

Die Viskosität der erfindungsgemässen Massen ist gegenüber dem reinen Glasionomerzement deutlich verbessert. Gerade die Verbesserung ist für die praktische Anwendung von grosser Bedeutung, da nur eine ausreichende Fliessfähigkeit ein homogenes Einfüllen in den schmalen Wurzelkanal ermöglicht.

## Patentansprüche

1. Verformbare Masse, bestehend aus
   (A) 25 bis 80 Gew.-% eines Glasionomerzementes, enthaltend
      (a) ein Aluminiumfluorosilikatglas,
      (b) mindestens eine polymere Polysäure mit einem mittleren Molekulargewicht > 500,
      (c) Wasser und
      (d) gegebenenfalls ein chelatbildendes Mittel, und
   (B) 20 bis 75 Gew.-% eines in der wässrigen polymeren Polysäure und in der den Chelatbildner enthaltenden Lösung im wesentlichen unlöslichen Fluorids und/oder Oxids und/oder Mischfluorids und-/oder Mischoxids von Schwermetallelementen.

2. Verformbare Masse nach Anspruch 1, dadurch gekennzeichnet, dass die Komponente (A) in einer Menge von 35 bis 65 Gew.-%, vorzugsweise 50 bis 65 Gew.-%, und Komponente (B) in einer Menge von 35 bis 65 Gew.-%, vorzugsweise 50 bis 65 Gew.-%, vorliegen.

3. Verformbare Masse nach den Ansprüchen 1 und 2, dadurch gekennzeichnet, dass das Schwermetallelement ein seltenes Erdmetall oder ein Metall der 6. Nebengruppe des Periodensystems ist.

4. Verformbare Masse nach den Ansprüchen 1 bis 3, dadurch gekennzeichnet, dass die Komponente (B) Yttriumfluorid, Zirkondioxid, Lanthanoxid, Calciumwolframat oder Strontiumwolframat, vorzugsweise Calciumwolframat, ist.

5. Verformbare Masse nach den Ansprüchen 1 bis 4, dadurch gekennzeichnet, dass sie in mindestens zwei räumlich voneinander getrennten Teilmassen vorliegt.

6. Verformbare Masse nach Anspruch 5, dadurch gekennzeichnet, dass mindestens eine Teilmasse in fester, vorzugsweise pulverförmiger Form und mindestens eine andere Teilmasse in flüssiger oder pastöser Form vorliegen.

7. Verformbare Masse nach Anspruch 6, dadurch gekennzeichnet, dass die feste Teilmasse die Bestandteile (A)(a) und (B) und die flüssige Teilmasse die Bestandteile (A)(b), (A)(c) und gegebenenfalls (A)(d) enthalten.

8. Verformbare Masse nach Anspruch 6, dadurch gekennzeichnet, dass die feste Teilmasse die Bestandteile (A)(a), (A)(b) und B und die flüssige Teilmasse die Bestandteile (A)(c) und gegebenenfalls (A)(d) enthalten.

9. Verformbare Masse nach Anspruch 6, dadurch gekennzeichnet, dass die feste Teilmasse die Bestandteile (A)(a), (A)(b), gegebenenfalls (A)(d), und B und die flüssige Teilmasse den Bestandteil (A)(c) enthalten.

10. Verwendung der verformbaren Masse nach den Ansprüchen 1 bis 9 als Füllungsmaterial für Zahnwurzelkanäle.

## Claims

1. Formable composition comprising
   (A) 25 to 80 wt-% of a glass ionomer cement, containing
      (a) an aluminium fluorosilicate glass,

(b) at least one polymeric polyacid with an average molecular weight > 500,

(c) water and

(d) optionally a chelate-forming agent and

(B) 20 to 75 wt-% of a fluoride and/or oxide and/or mixed fluoride and/or mixed oxide of heavy metal elements which is/are essentially insoluble in the aqueous polymeric polyacid and in the solution containing the chelating agent.

2. Formable composition according to claim 1, characterized in that the component (A) is present in a quantity of 35 to 65 wt-%, preferably 50 to 65 wt-%, and component (B) in a quantitiy of 35 to 65 wt-%, preferably 50 to 65 wt-%.

3. Formable composition according to claims 1 and 2, characterized in that the heavy metal element is a rare earth metal or a metal of the sixth sub-group of the periodic system.

4. Formable composition according to claims 1 to 3, chararacterized in that the component (B) is yttrium fluoride, zirconium dioxide, lanthanum oxide, calcium tungstate or strontium tungstate, preferably calcium tungstate.

5. Formable composition according to claims 1 to 4, characterized in that it is present in at least two part-compositions spatially separated from each other.

6. Formable composition according to claim 5, characterized in that at least one part-composition is present in solid, preferably powder form and at least one other part-composition in liquid or paste form.

7. Formable composition according to claim 6, characterized in that the solid part-composition contains the constituents (A) (a) and (B), and the liquid part-composition the constituents (A) (b), (A) (c) and optionally (A) (d).

8. Formable composition according to claim 6, characterized in that the solid part-composition contains the constituents (A) (a), (A) (b) and B, and the liquid part-composition the constituents (A) (c) and optionally (A) (d).

9. Formable composition according to claim 6, characterized in that the solid part-composition contains the constituents (A) (a), (A) (b), optionally (A) (d) and B, and the liquid part-composition the constituent (A) (C).

10. Use of the formable composition according to claims 1 to 9 as filling material for dental root canals.


## Revendications

1. Composition déformable, formée de :

(A) 25 à 80 % en poids d'un ciment d'ionomère de verre, contenant :

(a) un verre de fluorosilicate d'aluminium,

(b) au moins un polyacide polymère d'un poids moléculaire moyen > 500,

(c) de l'eau, et

(d) éventuellement un agent de chélation, et

(B) 20 à 75 % en poids d'un fluorure et/ou d'un oxyde et/ou d'un fluorure mixte et/ou d'un oxyde mixte d'éléments de métaux lourds, essentiellement insoluble dans le polyacide polymère aqueux et dans la solution contenant l'agent de chélation.

2. Composition déformable selon la revendication 1, caractérisée en ce que le composant (A) est présent en une quantité valant de 35 à 65 % en poids, de préférence de 50 à 65 % en poids, et le composant (B) est présent en une quantité valant de 35 à 65 % en poids, de préférence de 50 à 65 % en poids.

3. Composition déformable selon les revendications 1 et 2, caractérisée en ce que l'élément de métal lourd est un métal des terres rares ou un métal du sous-groupe 6 du système périodique.

4. Composition déformable selon les revendications 1 à 3, caractérisée en ce que le composant (B) est le fluorure d'yttrium, le dioxyde de zirconium, l'oxyde de lanthane, le tungstate de calcium ou le tungstate de strontium, de préférence le tungstate de calcium.

5. Composition déformable selon les revendications 1 à 4, caractérisée en ce qu'elle se présente sous la forme d'au moins deux compositions partielles séparées dans l'espace.

6. Composition déformable selon la revendication 5, caractérisée en ce qu'au moins une composition partielle est à l'état solide, de préférence à l'état pulvérulent, et au moins une autre composition partielle est à l'état liquide ou pâteux.

7. Composition déformable selon la revendication 6, caractérisée en ce que la composition partielle solide contient les constituants (A)(a) et (B), et la composition partielle liquide contient les constituants (A)(b), (A)(c) et éventuellement (A)(d).

8. Composition déformable selon la revendication 6, caractérisée en ce que la composition partielle solide contient les constituants (A)(a), (A)(b) et (B), et la composition partielle liquide contient les constituants (A)(c) et éventuellement (A)(d).

9. Composition déformable selon la revendication 6, caractérisée en ce que la composition partielle solide contient les constituants (A)(a), (A)(b), éventuellement (A)(d), et (B), et la composition partielle liquide contient le constituant (A)(c).

10. Utilisation de la composition déformable selon les revendications 1 à 9 comme matériau de remplissage des canaux radiculaires des dents.